# EUROPEAN PATENT APPLICATION

(11) **EP 4 487 850 A1**
(43) Date of publication of application: **08.01.2025**
(21) Application number: 23762878.9
(22) Date of filing: 28.02.2023
(51) Int. Cl.: A61K 31/4965, A61P 3/10, A61P 13/12

(54) **USE OF NITRONE COMPOUND OR PHARMACEUTICALLY ACCEPTABLE SALT THEREOF IN COMBINATION WITH SGLT-2 INHIBITOR**

(30) Priority: 02.03.2022 CN 202210205372; 29.07.2022 CN 202210905164
(71) Applicant: Guangzhou Magpie Pharmaceuticals Co., Ltd., Guangzhou, Guangdong 510005 (CN)
(72) Inventor: WANG, Yuqiang, Guangzhou, Guangdong 510005 (CN); JING, Mei, Guangzhou, Guangdong 510005 (CN); ZHANG, Zaijun, Guangzhou, Guangdong 510005 (CN); ZHANG, Gaoxiao, Guangzhou, Guangdong 510005 (CN); SUN, Yewei, Guangzhou, Guangdong 510005 (CN); LIU, Wei, Guangzhou, Guangdong 510005 (CN)
(74) Representative: Uexküll & Stolberg
(86) International application number: PCT/CN2023/078809
(87) International publication number: WO 2023/165479

(57) **Abstract**

The present invention provides a combination therapy of a nitrone compound or a pharmaceutically acceptable salt thereof with a SGLT-2 inhibitor. Research found that the nitrone compounds TBN and TN-2, when used in combination with a SGLT-2 inhibitor, can synergistically lower blood sugar and also reduce the 24-hour urinary microalbumin and urinary albumin creatinine ratio in db/db mice, demonstrating a protective effect on the kidneys. The inventor also unexpectedly discovered that the combination of the nitrone compound with the SGLT-2 inhibitor can reduce the risk of urinary and reproductive tract infections caused by SGLT-2 inhibitors.

## Description

### FIELD OF THE INVENTION

The present invention relates generally to the field of medicine and, more particularly, to combination therapy of medications, specifically to the combination therapy of a nitrone compound or a pharmaceutically acceptable salt thereof with a SGLT-2 inhibitor.

### BACKGROUND OF THE INVENTION

Diabetes is a group of metabolic diseases characterized by hyperglycemia. According to the data released by the International Diabetes Alliance in 2019, there are 463 million diabetes patients in the world. It is estimated that there will be 578 million diabetes patients in the world in 2030, and the number of diabetes patients will further increase to 700 million by 2045. China has the largest number of patients with diabetes in the world, and the number of patients with type II diabetes has exceeded 120 million. Long term elevated blood sugar can lead to damage to large blood vessels and microvasculature, endangering tissues or organs such as the heart, brain, kidneys, peripheral nerves, eyes, feet, etc. According to the statistics of the World Health Organization, diabetes has more than 100 complications, which is the disease with the most known complications. Clinical data show that 30%~40% of patients with diabetes will have at least one complication 10 years after the onset of diabetes. Traditional hypoglycemic drugs, such as insulin, biguanides, thiazolidinediones, and proinsulin secretion drugs, α-glucosidase inhibitors, DPP-4 enzyme inhibitors, are mostly limited to lowering blood sugar and cannot bring benefits in terms of complications to patients.

Unlike traditional hypoglycemic drugs, SGLT-2 inhibitors (sodium dependent glucose transporters 2 inhibitors) lower blood sugar by inhibiting the re-absorption of glucose by the kidneys, allowing excess glucose to be excreted from urine. SGLT-2 inhibitors have potential protective effect on renal function in patients with mild to moderate renal insufficiency, can improve proteinuria in patients with diabetes, and delay the progress of kidney disease. Clinical studies have shown that SGLT-2 inhibitors exhibit a certain degree of acute kidney injury within 1-2 months of administration, then gradually recover and show renal protective effects one year later (See David Z.I. Cherney, Francesco Cosentino,2 Samuel Dagogo-Jack, et al. Ertugliflozin and Slope of Chronic eGFR Prespecified Analyses from the Randomized VERTIS CV Trial, Clin J Am Soc Nephrol[J]. 2021 Sep; 16(9): 1345-1354).

SGLT-2 inhibitors can reduce blood sugar with kidney benefits, and thus in 2015, the American Diabetes Association (ADA) and The European Association for the Study of Diabetes (EASD) both recommended SGLT-2 inhibitors as second-line and third-line drugs for type II diabetes, and can be used in combination with metformin or other hypoglycemic drugs.

Although SGLT-2 inhibitors in clinical use not only reduce blood glucose, but also have a renal protection mechanism, which provides new possibilities for the clinical treatment of diabetes complications, SGLT-2 inhibitors increase urinary glucose excretion, increase the glucose concentration in the genitourinary tract, and will lead to increased opportunities for bacterial and fungal infections.

### SUMMARY OF THE INVENTION

The present invention is directed to provide a composition, a kit and a combination of active agents, and use thereof in manufacture of a medicament for prevention and treatment of diabetes and their complications. Research has shown that the combination of the nitrone compounds of the present invention or their pharmaceutically acceptable salts with SGLT-2 inhibitors can enhance renal protection and further reduce IL-6 levels. The results suggest that the combination use can reduce the risk of urinary and reproductive system infections caused by SGLT-2 inhibitors, which has important clinical significance.

Therefore, in a first aspect, the present invention provides a composition comprising:
(a) a first active agent, which is a nitrone compound or a pharmaceutically acceptable salt thereof; and
(b) a second active agent, which is an SGLT-2 inhibitor;
wherein, the nitrone compound has a structure of the following formula (I): 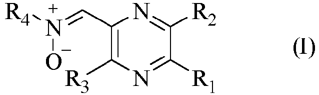 wherein:
R₁ is hydrogen, methyl, or 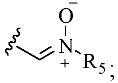
R₂ and R₃ are the same or different, each independently selected from hydrogen and C₁-C₆ alkyl; R₄ is sec-butyl, isobutyl, t-butyl, cyclopentyl or cyclohexyl, and R₅ is sec-butyl, isobutyl, t-butyl, or cyclopentyl.

Preferably, R₂ and R₃ are the same or different, each independently selected from hydrogen, methyl, ethyl, n-propyl, isopropyl, n-butyl, isobutyl, tert-butyl, and n-pentyl; R₁ is hydrogen, methyl, or

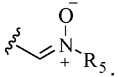

Further preferably, the nitrone compound has a structure of the following formula, TBN or TN-2:

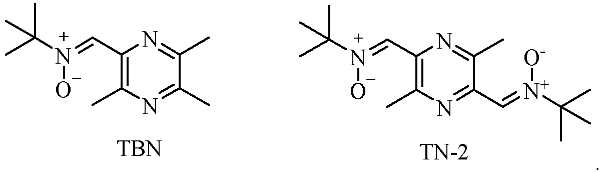

According to some embodiments of the present invention, the SGLT-2 inhibitor is selected from Dapagliflozin, Canagliflozin, Empagliflozin (Empag), Ipragliflozin, Luseogliflozin, Tofogliflozin, Ertugliflozin, Sergliflozin etabonate, Sotagliflozin, Rongliflozin L-Pyroglutamic Acid, Wangliflozin, Tagliflozin, Jiagliflozin, Henggliflozin, or a combination thereof.

In a second aspect, the present invention provides a pharmaceutical kit, comprising:
(a) a first preparation, comprising a nitrone compound or a pharmaceutically acceptable salt thereof;
(b) a second preparation, comprising a SGLT-2 inhibitor, which is selected from Dapagliflozin, Canagliflozin, Empagliflozin (Empag), Ipragliflozin, Luseogliflozin, Tofogliflozin, Ertugliflozin, Sergliflozin etabonate, Sotagliflozin, Rongliflozin L-Pyroglutamic Acid, Wangliflozin, Tagliflozin, Jiagliflozin, Henggliflozin, or a combination thereof; and
(c) instructions.

The pharmaceutical kid herein serves as a carrier for storing or transferring drugs.

In a third aspect, the present invention provides a pharmaceutical composition, which comprises:
(a) a first active agent, comprising a nitrone compounds or a pharmaceutically acceptable salt thereof;
(b) a second agent active agent, comprising a SGLT-2 inhibitor, which is selected from Dapagliflozin, Canagliflozin, Empagliflozin (Empag), Ipragliflozin, Luseogliflozin, Tofogliflozin, Ertugliflozin, Sergliflozin etabonate, Sotagliflozin, Rongliflozin L-Pyroglutamic Acid, Wangliflozin, Tagliflozin, Jiagliflozin, Henggliflozin, or a combination thereof; and
(c) a pharmaceutically acceptable carrier.

In a fourth aspect, the present invention provides a combination therapy.

The present invention provides a combination of a nitrone compound or a pharmaceutically acceptable salt thereof with a SGLT-2 inhibitor for the prevention and treatment of chronic kidney disease. Furthermore, the nitrone compound or a pharmaceutically acceptable salt thereof is used in combination with a SGLT-2 inhibitor in the manufacture of a medicament for the prevention and treatment of diabetes or/and a complication thereof.

The complication of diabetes is one or more of diabetes complicated with hypertension, diabetic kidney disease, diabetes complicated with reproductive tract infection, diabetic retinopathy, diabetic cataract, and diabetic neuropathy.

The medicament comprising a SGLT-2 inhibitor includes a SGLT-2 inhibitor, and may also include other hypoglycemic agent or/and antihypertensive agent. The other hypoglycemic drug is selected from insulin, biguanides, thiazolidinediones, insulin secretagogue, α-glucosidase inhibitor, DPP-4 enzyme inhibitor, glucosidase inhibitor, and dipeptidyl peptidase inhibitor, or a combination thereof; and the antihypertensive drug is selected from angiotensin receptor blockers and angiotensin converting enzyme inhibitors, or a combination thereof.

Preferably, the biguanides are selected from metformin, phenformin, and succinbiguanide, or a combination thereof; the insulin secretagogue is selected from toluenesulfonylurea, hexylurea acetate, tolasulfonylurea, chlorosulfonylurea, glipizide, glibenclamide, glimepiride, gliclazide, gliquidone, regagliptin, nateglinide, or a combination thereof; the thiazolidinediones are selected from traglitazone, rosiglitazone, pioglitazone, cyclogliptine, or a combination thereof; the α-glucosidase inhibitor is selected from Miglitaxel, Acarbose, Voglibose, or a combination thereof; the DPP-4 enzyme inhibitor is selected from sitagliptin, sagagliptin, vitagliptin, ligagliptin, agliptin, or a combination thereof.

The combined administration herein can be simultaneous administration or separate administration.

The present invention also provides use of the nitrone compound or a pharmaceutically acceptable salt thereof in the manufacture of a medicament for the prevention and/or treatment of reproductive tract infections caused by SGLT-2 inhibitors.

The dosage of the SGLT-2 inhibitor described in the present invention can be determined by clinical physicians based on the severity of the disease, disease response, any treatment-related toxicity, patient age, patient weight, and health status. The specific route of administration and frequency of administration can be determined according to the conventional route of administration of the drugs in the art. For example, the dosage of SGLT-2 inhibitors described herein can be based on SGLT-2 inhibitors ranging from 0.5mg-500 mg/person/day. The preferred dosage range for the SGLT-2 inhibitor of Engagliflozin is 1 mg -100 mg/day, more preferrably 1 mg -50 mg/day, such as 1, 2.5, 5, 7.5, 10, 12.5, 15, 20, 25, or 50 mg/day. The preferred dosage range for SGLT-2 inhibitor of Canagliflozin is 50 mg-500 mg/day, more preferrably 50 mg-300 mg/day, such as 50, 100, 150, 200, 250, or 300 mg/day. The preferred dosage range for SGLT-2 inhibitor of Dapagliflozin is 1 mg -200 mg/day, more preferrably 1 mg -100 mg/day, such as 1, 2.5, 5, 7.5, 10, 12.5, 15, 20, 25, 50 or 100 mg/day. The specific dosage can be determined based on the severity of the disease, disease response, any treatment-related toxicity, patient age, and health conditions. The specific route of administration and frequency of administration can follow the conventional route of administration of SGLT-2 inhibitors, such as once or multiple times a day.

The dosage of the nitrone compound or a pharmaceutically acceptable salt thereof according to the present invention can be calculated as 100-3000 mg/person/time according to the dosage of tetramethylpyrazine nitrone (TBN), such as 300-1200 mg/person/time (1-3 times a day). The preferred dose range for TBN is 100 mg -3000 mg/day, more preferably 300-2400 mg/day, such as 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, or 3000mg/day. The specific dosage can be determined based on the severity of the disease, disease response, any treatment-related toxicity, patient age, and health conditions. The specific route of administration and frequency of administration can be based on the conventional route of administration of TBN, such as once or multiple times a day.

According to some embodiments of the present invention, the nitrone compound, as an active pharmaceutical agent, can be formulated into a pharmaceutical composition using a pharmaceutically acceptable carrier. The pharmaceutical composition is any composition suitable for oral, sublingual, local inhalation (nasal spray), rectal, intramuscular, dermal, subcutaneous or intravenous administration. The amount of the composition required for treatment will vary depending on the route of administration, the nature of the treated condition, patient age, and health conditions, ultimately determined by the participating clinical physicians. The required dose can be provided in single or split doses (with appropriate intervals), for example, two, three, or more times a day to achieve or meet treatment needs.

The term "pharmaceutically acceptable carrier" used herein refers to a substance that does not interfere with the physiological effects of the nitrone compound and is non-toxic to mammals including humans. In an embodiment, the pharmaceutical composition is prepared with tetramethylpyrazine nitrone (TBN) and a pharmaceutically acceptable carrier. The pharmaceutical composition can be prepared in a form of, including but not limited to, solids, capsules, pills, suppositories, liquids (such as injections), oils, emulsions, spray, gel, aerosols, inhalants, or patches.

Research found that the nitrone compounds TBN and TN-2, when used in combination with a SGLT-2 inhibitor, can synergistically lower blood sugar and also reduce the 24-hour urinary microalbumin and urinary albumin creatinine ratio in db/db mice, demonstrating a protective effect on the kidneys. The clinical trial results also indicate that the combination use of the nitrone compound of the present invention with a SGLT-2 inhibitor can significantly enhance the extent of reduction in UACR, demonstrating a synergistic renal protective effect.

The Chinese Expert Consensus (2019 version) and the Chinese Diabetic Kidney Disease Prevention and Treatment Clinical Guidelines (2019 version) point out that SGLT-2 inhibitors have more advantages in reducing blood sugar than other oral hypoglycemic drugs, and the longer the treatment time, the more obvious this advantage is, but it will increase the risk of urinary tract and genital infection, mainly because this kind of drugs increase the excretion of urine sugar, but also increase the reproduction of bacteria due to high urine sugar. Regarding the signal intensity comparison of urinary tract infections, dapagliflozin > canagliflozin > empagliflozin (see Chen Qiying, Li Yimin, and Zhang Yin, Analysis of adverse reactions of sodium glucose co transporter-2 inhibitors based on FAERS [J]. Chinese Journal of Modern Applied Pharmacy, 2021, 38 (14): 1729-1733). Oversee studies also show that diabetes can increase the risk of genital infection, and SGLT-2 inhibitors, a new hypoglycemic drug, are more prone to genitourinary infections due to their unique hypoglycemic mechanism (See Li D, Wang T, Shen S, Fang Z, Dong Y, and Tang H, Urinary tract and genital infections in patients with type II diabetes treated with sodium-glucose co-transporter 2 inhibitors: A meta-analysis of randomized controlled trials. Diabetes Obes Metab. 2017 Mar;19(3):348-355). The risk of urinary tract infections associated with SGLT-2 inhibitors is significantly higher in female patients than in male patients. (See Shen J, Yang J, and Zhao B,A Survey of the FDA's Adverse Event Reporting System Database Concerning Urogenital Tract Infections and Sodium Glucose Cotransporter-2 Inhibitor Use. Diabetes Ther. 2019 Jun; 10(3):1043-1050).

Urinary tract infection is an inflammatory disease caused by the growth and reproduction of various pathogenic microorganisms in the urinary tract. Clean midstream urine culture is an important basis for diagnosing urinary tract infections. However, urine bacterial culture takes about 3-4 days, which cannot provide timely diagnosis and treatment basis for clinical practice. In addition, inaccurate results can be caused by inadequate colony count and susceptibility to contamination during the culture process. Therefore, clinical practice requires more specific and sensitive indicators to diagnose urinary tract infections.

Numerous studies both domestically and internationally have shown a high correlation between urinary and reproductive tract infections and levels of interleukin-6 (IL-6), which can serve as a biomarker for early diagnosis of urinary and reproductive tract infections (See Rashid MH, Sparrow NA, Anwar F, Guidry G, Covarrubias AE, Pang H, Bogguri C, Karumanchi SA, and Lahiri S, Interleukin-6 mediates delirium-like phenotypes in a murine model of urinary tract infection. J. Neuroinflammation. 2021 Oct 28;18(1):247; Peng Xuan, and Ke Guibao et al., Changes and clinical significance of serum and peptide levels, IL-6, and PCT in patients with urinary tract infections [J], Practical Clinical Medicine, 2017, 01: 13-15; Sun Yuyan, and Ding Sanzhen, et al., Urine bacterial distribution in female patients with type II diabetes and its correlation with serum IL-6 [J], Chinese Journal of Microbiology, 2021, 33 (10): 1170-1175). The inventors unexpectedly discovered that the nitrone compound (such as TBN, TN-2), alone or in combination with a SGLT-2 inhibitor (such as Entegliflozin), can significantly reduce the content of IL-6 in the serum of model animals. The combination use of the nitrone compound with a SGLT-2 inhibitor can reduce the risk of urinary and reproductive tract infections caused by SGLT-2 inhibitors. The nitrone compound of the invention can not only be used as an antagonist of IL-6 in serum, but also can be used for the treatment of diabetes combined with reproductive tract infection.

In an embodiment, the present invention provides a use of the nitrone compound or a pharmaceutically acceptable salt thereof in manufacture of a medicament for the prevention or treatment of chronic kidney disease, wherein the medicament is an oral drug formulation comprising essentially TBN, which is administered in unit dose form, containing a therapeutically effective amount of TBN or a pharmaceutically acceptable salt thereof, and the therapeutically effective amount is of approximately 600-2400 mg per day, which can effectively treat, prevent, and reduce the risk of chronic kidney disease, delay its occurrence, and/or delay its progression.

In other embodiments, the therapeutically effective amount can also be about 600-1200 mg per day, about 1200-2400 mg per day, about 600 mg per day, about 1200 mg per day, or about 2400 mg per day.

In other embodiments of the present invention provides an oral drug formulation for the prevention or treatment of chronic kidney disease, wherein the oral drug formulation includes a SGLT-2 inhibitor. The SGLT-2 inhibitor is selected from Dapagliflozin, Canagliflozin, Empagliflozin (Empag), Ipragliflozin, Luseogliflozin, Tofogliflozin, Ertugliflozin, Sergliflozin etabonate, Sotagliflozin, Rongliflozin L-Pyroglutamic Acid, Wangliflozin, Tagliflozin, Jiagliflozin, Henggliflozin, or a combination thereof.

In some embodiments of the invention, the complications of the chronic kidney disease are one or more of diabetes complicated with chronic kidney disease and chronic kidney disease complicated with infection.

### BRIEF DESCRIPTION OF THE DRAWINGS

Figure 1 shows the 24-hour content of urinary microalbumin of different groups of mice in Example 1;
Figure 2 shows the levels of blood glucose of different groups of mice in Example 1;
Figure 3 shows the levels of IL-6 in the serum of different groups of mice in Example 1;
Figure 4 shows the levels of IL-1β in the serum of different groups of mice in Example 1;
Figure 5 shows the levels of urea nitrogen in the serum of different groups of mice in Example 1;
Figure 6 shows the levels of triglycerides in the serum of different groups of mice in Example 1;
Figure 7 shows the blood glucose levels of different groups of mice in Example 2.

### DETAILED DESCRIPTION OF THE EMBODIMENTS

Some specific embodiments or examples of the present invention will be described below. It should be pointed out that the following embodiments or examples are only for further explanation of the present invention and cannot be understood as limiting the scope of the invention. Unless otherwise specified, all portions of the present invention are by weight, and all percentages are by mass.

In the embodiments of the present invention, "wt/wt" refers to wild-type mice, "i.g" refers to intragastric administration, and "po" refers to oral administration.

### Example 1. Grouping and pharmacodynamics study of TBN and TN-2 alone and their combination with Empagliflozin in spontaneous type II diabetes model mice

Db/db mice are type II diabetes mice caused by leptin receptor gene defect located on chromosome 4. They began to drink, eat, urinate and be obese at the age of 4 weeks. With the increase of age, they have obvious characteristics of hyperglycemia, hyperlipidemia and insulin resistance. Their pathogenesis is very similar to that of type II diabetes patients. With the aggravation of diabetes, db/db mice have a series of complications. The main pathological changes of kidney tissue are dilation of glomerular mesangial interstitium, thickening of glomerular basement membrane, excessive accumulation of extracellular matrix, etc. It is a good model to study the early morphological changes of DKD kidney.

### (1) Animal grouping and administration, as shown in Table 1

**Table 1 Grouping and specific administration of experimental mice**

| **Groups** | **Route of administration** |
|---|---|
| Ctrl (wt/wt, saline, twice/day) | i.g. |
| Model (db/db, saline, twice/day) | i.g. |
| TBN (10 mg/kg, twice/day) | i.g. |
| TBN (30 mg/kg, twice/day) | i.g. |
| TN-2 (10 mg/kg, twice/day) | i.g. |
| Empagliflozin (2.5 mg/kg, twice/day) | i.g. |
| Empagliflozin (5 mg/kg, twice/day) | i.g. |
| TBN (10 mg/kg, twice/day)+Empagliflozin (2.5 mg/kg, twice/day) | i.g. |
| TBN (30 mg/kg, twice/day)+Empagliflozin (2.5 mg/kg, twice/day) | i.g. |
| TN-2 (10 mg/kg, twice/day)+Empagliflozin (2.5 mg/kg, twice/day) | i.g. |

6-week-old db/db mice were given adaptive feeding for 1 week (i.e. 7 weeks), with TBN and TN-2 administered separately and in combination with Empagliflozin twice a day (fixed at 9:00-10:00 am and 3:00-4:00 pm, with an interval of 6 hours), for a continuous 6 weeks of administration.

### (2) Pharmacological study

### Blood glucose testing

During the experiment, blood was collected from the tail vein of mice before the start of administration (7 weeks old) to measure their blood glucose levels; After the completion of administration (at 13 weeks of mouse age, i.e. the end of the experiment), serum was taken and the blood glucose levels of each group of mice were measured using a fully automated biochemical analyzer.

### Detection of 24-hour urinary microalbumin

Db /db mice were tested for 24-hour urinary microalbumin at 4 and 6 weeks after drug treatment. Each group of mice was placed in a metabolic cage, drinking water freely, and 24-hour urine samples were collected. After centrifugation and precipitation, the levels of microalbuminuria in the 24-hour urine of each group of mice were detected using an ELISA kit.

### Detection of the level of IL-6 and IL-1β in serum

After 6 weeks of administration, the whole blood was left to stand at room temperature for 1 hour. Then, it was transferred to a centrifuge and centrifuged at 3000 rpm for 10 minutes. The upper layer of serum was taken out and placed in an EP tube and stored in a -20 °C freezer for later use. Detection the level of IL-6 and IL-1β in serum of mice in each group using ELISA kit.

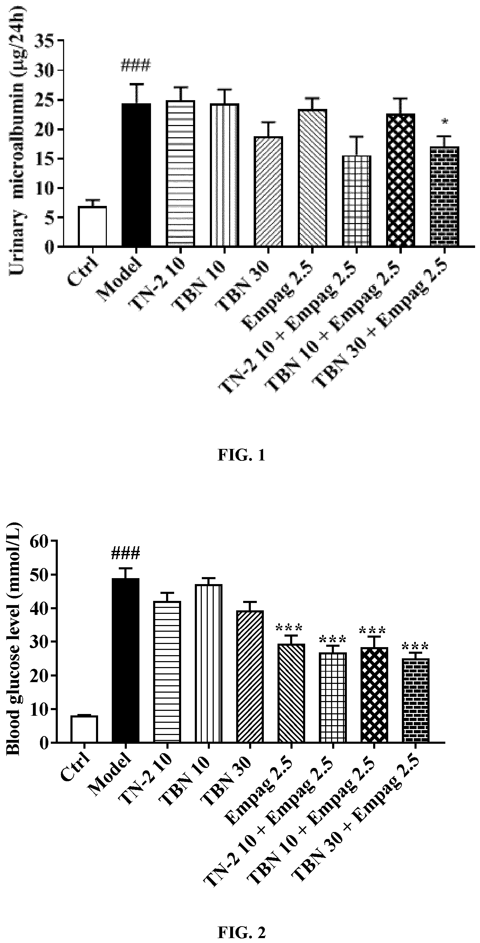

The 24-hour urinary microalbumin levels of mice treated with TBN, TN-2, and Empagliflozin alone or in combination are shown in Figure 1. The combined administration of TBN and Empagliflozin, as well as TN-2 and Empagliflozin, further reduced the 24-hour urinary microalbumin level compared to the group treated alone.

The blood glucose level of mice treated with TBN, TN-2, and Empagliflozin alone or in combination is shown in Figure 2. The combined administration of TBN and Empagliflozin, as well as TN-2 and Empagliflozin, can effectively lower the level of blood glucose.

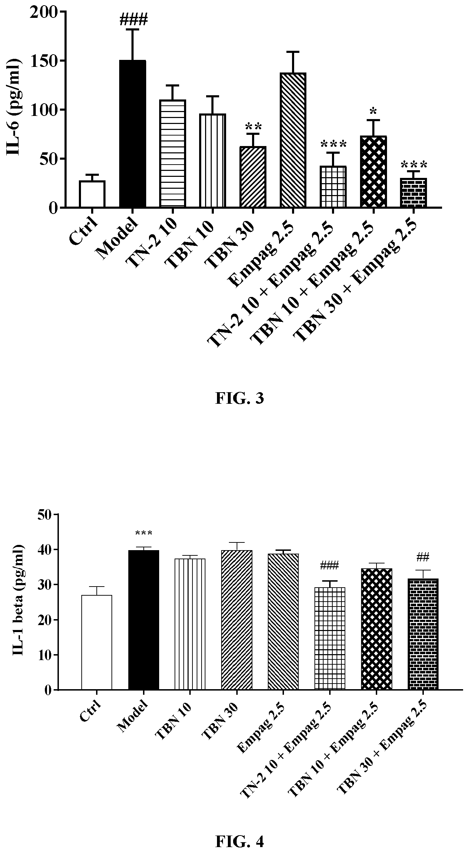

The levels of IL-6 in the serum of mice treated with TBN, TN-2, and Empagliflozin alone or in combination are shown in Figure 3. The combined administration of TBN and Empagliflozin, as well as TN-2 and Empagliflozin, can significantly reduce the level of IL-6 in serum IL-6.

The levels of IL-1β in the serum of mice treated with TBN, TN-2, and Empagliflozin alone or in combination are shown in Figure 4. The combined administration of TBN and Empagliflozin, as well as TN-2 and Empagliflozin, can significantly reduce the level of IL-1β in serum.

Values are shown as means ± SEM. ^{###}*P* < 0.001 vs db/m group, *P < 0.05, ***P* < 0.01, and ****P* < 0.001 vs db/db group, *^{&}P* < 0.05 and *^{&&&}P* < 0.001 vs Empagliflozin 2.5 mg/kg group.

The specific results of the percentage changes in the levels of blood glucose, urinary microalbumin, and serum IL-6 and IL-1β of TBN and Empagliflozin alone and in combination administered by gavage compared to the Model group are shown in Table 2.

**Table 2. Changes in the levels of IL-6 and IL-1β in blood glucose, urinary microalbumin and serum**

| **Groups** | | **Changes in blood glucose** | **Changes in urinary microalbumin** | **Changes in IL-1β** | **Changes in IL-6** |
|---|---|---|---|---|---|
| TBN | 10 mg/kg | -0.80% | -0.61% | -5.88% | -36.43% |
| | 30 mg/kg | -17.13% | -23.09% | 0.20% | -58.34% |
| Empagliflozin | 2.5 mg/kg | -38.03% | -4.18% | -2.36% | -8.55% |
| | 5 mg/kg | -44.33% | -18.62% | -16.32% | -75.05% |
| TBN 10 mg/kg + Empagliflozin 2.5 mg/kg | | -40.43% | -7.65% | -13.06% | -51.33% |
| TBN 30 mg/kg + Empagliflozin 2.5 mg/kg | | -47.17% | -31.40% | -20.29% | -79.95% |

Table 2 clearly indicate that the combination of the nitrone compound TBN and the SGLT-2 inhibitor Empagliflozin in the present invention can enhance the extent of reduction of blood sugar, especially the blood sugar reduction in the TBN 10 mg/kg+Empagliflozin 2.5 mg/kg group is better than the sum of the TBN 10 mg/kg group and Empagliflozin 2.5 mg/kg group; After combination use, both the TBN 10 mg/kg+Empagliflozin 2.5 mg/kg group and the TBN 30 mg/kg+Empagliflozin 2.5 mg/kg group were able to enhance the extent of reduction in urinary protein, and the reduction in both groups was better than the sum of the TBN 10 mg/kg group and Empagliflozin 2.5 mg/kg group; After combination use, both the TBN 10 mg/kg+Empagliflozin 2.5 mg/kg group and the TBN 30 mg/kg+Empagliflozin 2.5 mg/kg group can enhance the extent of reduction in IL-1β levels. The extend of reduction in the levels of both groups was better than the sum of TBN 10 mg/kg group and Empagliflozin 2.5 mg/kg group; After combination use, both the TBN 10 mg/kg+Empagliflozin 2.5 mg/kg group and the TBN 30 mg/kg+Empagliflozin 2.5 mg/kg group were able to enhance the extent of reduction in IL-6, and the reduction in both groups was better than the sum of the TBN 10 mg/kg group and Empagliflozin 2.5 mg/kg group.

SGLT-2 inhibitors are used to lower blood sugar levels by increasing the excretion of urine sugar. However, high urine sugar also increases the proliferation of bacteria in the urinary and reproductive tract, leading to urinary tract infections and related inflammation. The combination use of the nitrone compounds and SGLT-2 inhibitors further reduces the level of IL-6, indicating that the nitrone compounds can reduce the risk of urinary and reproductive system infections caused by SGLT-2 inhibitor drugs, and have important clinical significance.

### Detection on the levels of urea nitrogen and triglycerides in the serum of db/db mice

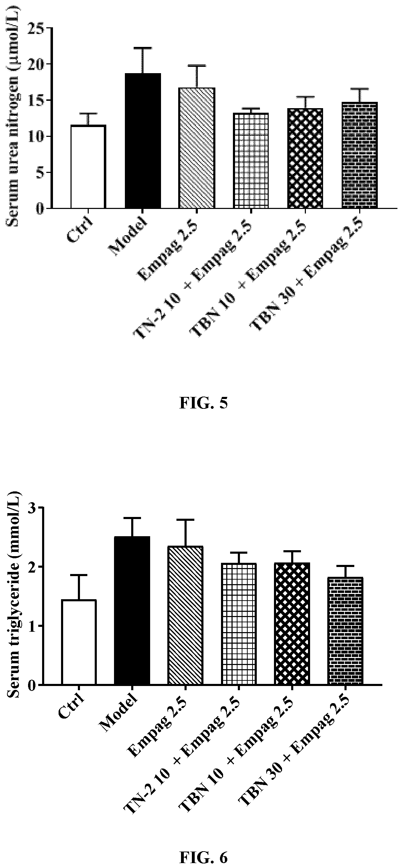

After anesthesia, blood was collected from the abdominal aorta of mice. After standing for 1 hour, it was centrifuged at 3000 rmp for 10 minutes and stored at -80 °C. The levels of urea nitrogen and triglycerides in serum were detected using a fully automated biochemical analyzer, and the results are shown in Figure 5-6.

### Example 2. Grouping and pharmacodynamic studies of TBN and Canagliflozin administered alone and in combination in DKD model mice

The grouping and administration of animals are shown in Table 3.

**Table 3. Grouping and administration of experimental mice**

| **Groups** | **Route of administration** |
|---|---|
| Ctrl (wt/wt, saline, twice/day) | i.g. |
| Model (db/db, saline, twice/day) | i.g. |
| TBN (30 mg/kg, twice/day) | i.g. |
| Canagliflozin (10 mg/kg, once/day) | i.g. |
| TBN (30 mg/kg, twice/day)+Canagliflozin (10 mg/kg, once/day) | i.g. |

Db/db mice of 6 weeks old were adaptively fed for 1 week (i.e., 7 weeks old) before administration. TBN and Canagliflozin were administered alone or in combination, with TBN administered twice a day (fixed at 9:00-10:00 am and 3:00-4:00 pm, with an interval of 6 hours) and Canagliflozin administered once a day (fixed at 9:00-10:00 am) for 8 weeks.

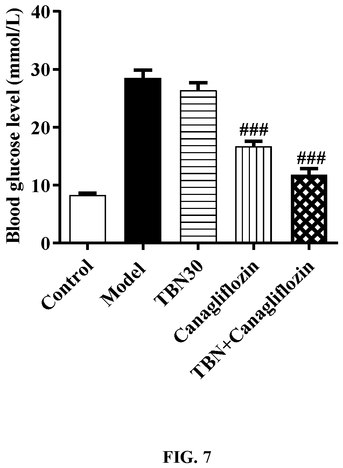

According to the method in Example 1, blood was taken from the tail vein of the mice before administration (7 weeks old) to measure their blood glucose levels; After the completion of administration (15 weeks old of mice, i.e. the end of the experiment), serum was taken and the blood glucose levels of each group of mice were measured using a fully automated biochemical analyzer (as shown in Figure 7). From Figure 7, it is evident that the combination of TBN and Canagliflozin can effectively reduce blood glucose levels.

### Example 3. Clinical trials on combination use of TBN and SGLT-2 inhibitor (SGLT2i)

The present invention adopts a test design of multicenter, randomized, double-blind, placebo-controlled parallel control.

Subjects of the experiments: Patients of type II diabetic kidney disease were taken as the subjects in the experiments (all signed the informed consent form). In at least 2 of the 3 morning urine in the screening period, the urinary microalbumin/creatinine ratio (UACR) is of ≥30 mg/g and <300 mg/g.

**Table 4. Grouping and Administration Status**

| | **Groups** | **Route of administration** |
|---|---|---|
| TBN | TBN (300 mg, twice/day) | po |
| | TBN (600 mg, twice/day) | po |
| | TBN (1200 mg, twice/day) | po |
| | Placebo (twice/day) | po |
| TBN+SGLT2i | TBN (300 mg, twice/day)+SGLT2i (once/day) | po |
| | TBN (600 mg, twice/day)+SGLT2i (once/day) | po |
| | TBN (1200 mg, twice/day)+SGLT2i (once/day) | po |
| | SGLT2i (once/day) | po |

The medication schedule: Administration of 180 consecutive days. In this example, SGLT2i is commercially available SGLT2i, selected from Engliflozin, Dapagliflozin, and Cargliflozin: Engliflozin once daily, 10 mg; Dapagliflozin once daily, 10 mg; Kagliflozin once daily, 100 mg. In the three subjects in the 300 mg TBN+SGLT2i group, one subject took Engliflozin and two subjects took Dapagliflozin; in the three subjects in the 600 mg TBN+SGLT2i group, all took Dapagliflozin; in the two subjects in the 1200 mg TBN+SGLT2i group, two subjects took Canagliflozin, one subject took Entegliflozin, and one subject took Dapagliflozin; in the SGLT2i group, one subject took Dapagliflozin.

Changes of UACR relative baseline in the TBN group (67 subjects) are shown in Table 5.

**Table 5. Percentage of changes in UACR relative baseline in TBN group**

| | TBN | | | Placebo (n=16) |
|---|---|---|---|---|
| | 300 mg (n=18) | 600 mg (n=19) | 1200 mg (n=14) | |
| Baseline mean UACR (SD) | 102.72 (83.42) | 94.52 (76.96) | 107.39 (79.54) | 101.57 (80.91) |
| Day 90 LS Mean | -1.48% | -43.32% | -37.08% | -11.12% |

The relative baseline changes of UACR in the combination therapy group (11 subjects) are shown in Table 6.

**Table 6. Percentage changes in UACR relative baseline in the combination therapy group**

| | TBN + SGLT2i | | | SGLT2i (n=1) |
|---|---|---|---|---|
| | 300 mg (n=3) | 600 mg (n=3) | 1200 mg (n=4) | |
| Baseline mean UACR (SD) | 132.76 (107.39) | 132.03 (27.30) | 111.27 (52.09) | 36.70 (-) |
| Day 90 LS Mean | 8.88% | -91.43% | -26.67% | 79.20% |
| Day 180 LS Mean | 31.81% | -118.9% | -21.38% | 188.6% |

The results in Tables 5 and 6 indicate that the combination use of the nitrone compounds TBN and SGLT2i in the present invention for 90 days/180 days significantly enhance the extent of reduction in UACR compared to the use of TBN or SGLT2i alone, achieving a synergistic effect.

## Claims

1. A composition, **characterized in that** the composition comprises:
a first active agent, which is a nitrone compound or a pharmaceutically acceptable salt thereof; and
a second active agent, which is an SGLT-2 inhibitor;
wherein, the nitrone compound has a structure of the following formula (I): 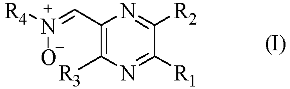 wherein:
R₁ is hydrogen, methyl or 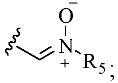
R₂ and R₃ are the same or different, each independently selected from hydrogen and C₁-C₆ alkyl; R₄ is sec-butyl, isobutyl, t-butyl, cyclopentyl or cyclohexyl, and R₅ is sec-butyl, isobutyl, t-butyl, or cyclopentyl;
preferably, R₂ and R₃ are the same or different, each independently selected from hydrogen, methyl, ethyl, n-propyl, isopropyl, n-butyl, isobutyl, tert-butyl, and n-pentyl; R₁ is hydrogen, methyl, or 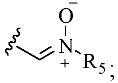
more preferably, the nitrone compound has a structure of the following formula, TBN or TN-2:

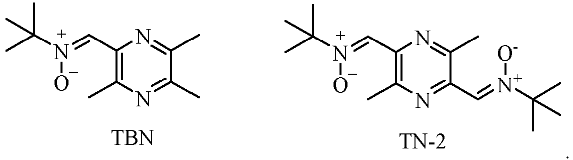

2. The composition of claim 1, wherein the SGLT-2 inhibitor the SGLT-2 inhibitor is selected from Dapagliflozin, Canagliflozin, Empagliflozin (Empag), Ipragliflozin, Luseogliflozin, Tofogliflozin, Ertugliflozin, Sergliflozin etabonate, Sotagliflozin, Rongliflozin L-Pyroglutamic Acid, Wangliflozin, Tagliflozin, Jiagliflozin, Henggliflozin, or a combination thereof.

3. A pharmaceutical kit, comprising:
a first preparation, comprising a therapeutically effective amount of a first active agent of claim 1;
a second preparation, comprising a therapeutically effective amount of a second active agent of claim 1; wherein the SGLT-2 inhibitor is selected from Dapagliflozin, Canagliflozin, Empagliflozin (Empag), Ipragliflozin, Luseogliflozin, Tofogliflozin, Ertugliflozin, Sergliflozin etabonate, Sotagliflozin, Rongliflozin L-Pyroglutamic Acid, Wangliflozin, Tagliflozin, Jiagliflozin, Henggliflozin, or a combination thereof; and
instructions.

4. A pharmaceutical composition, **characterized in that** the pharmaceutical composition comprises:
a therapeutically effective amount of a first active agent of claim 1;
a second preparation, comprising a therapeutically effective amount of a second active agent of claim 1; wherein the SGLT-2 inhibitor is selected from Dapagliflozin, Canagliflozin, Empagliflozin (Empag), Ipragliflozin, Luseogliflozin, Tofogliflozin, Ertugliflozin, Sergliflozin etabonate, Sotagliflozin, Rongliflozin L-Pyroglutamic Acid, Wangliflozin, Tagliflozin, Jiagliflozin, Henggliflozin, or a combination thereof; and
a pharmaceutically acceptable carrier.

5. Use of the composition of claim 1 in manufacture of a medicament, wherein the nitrone compound or a pharmaceutically acceptable salt is used in combination with an agent containing a SGLT-2 inhibitor, and the medicament is used for prevention and treatment of diabetes and a complication thereof.

6. The use of claim 5, wherein the complication of diabetes is one or more of diabetes complicated with hypertension, diabetic kidney disease, diabetes complicated with reproductive tract infection, diabetic retinopathy, diabetic cataract, and diabetic neuropathy.

7. The use of claim 5 or 6, wherein the agent comprising the SGLT-2 inhibitor further comprises a hypoglycemic agent and/or an antihypertensive agent;
wherein, the hypoglycemic drug is selected from insulin, biguanides, thiazolidinediones, insulin secretagogue, α-glucosidase inhibitor, DPP-4 enzyme inhibitor, glucosidase inhibitor, and dipeptidyl peptidase inhibitor, or a combination thereof; preferably, the biguanides are selected from metformin, phenformin, and succinbiguanide, or a combination thereof; the insulin secretagogue is selected from toluenesulfonylurea, hexylurea acetate, tolasulfonylurea, chlorosulfonylurea, glipizide, glibenclamide, glimepiride, gliclazide, gliquidone, regagliptin, nateglinide, or a combination thereof; the thiazolidinediones are selected from traglitazone, rosiglitazone, pioglitazone, cyclogliptine, or a combination thereof; the α-glucosidase inhibitor is selected from Miglitaxel, Acarbose, Voglibose, or a combination thereof; the DPP-4 enzyme inhibitor is selected from sitagliptin, sagagliptin, vitagliptin, ligagliptin, agliptin, or a combination thereof;
wherein, the antihypertensive drug is selected from angiotensin receptor blockers and angiotensin converting enzyme inhibitors, or a combination thereof.

8. Use of a nitrone compound or a pharmaceutically acceptable salt thereof in manufacture of a medicament for the prevention and/or treatment of reproductive tract infections caused by SGLT-2 inhibitors;
wherein, the nitrone compound has a structure of the following formula (I): 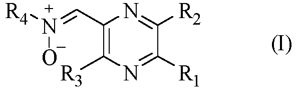 wherein:
R₁ is hydrogen, methyl, or 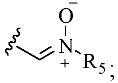
R₂ and R₃ are the same or different, each independently selected from hydrogen and C₁-C₆ alkyl; R₄ is sec-butyl, isobutyl, t-butyl, cyclopentyl or cyclohexyl, and R₅ is sec-butyl, isobutyl, t-butyl, or cyclopentyl;
preferably, R₂ and R₃ are the same or different, each independently selected from hydrogen, methyl, ethyl, n-propyl, isopropyl, n-butyl, isobutyl, tert-butyl, and n-pentyl; R₁ is hydrogen, methyl, or 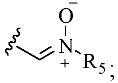
more preferably, the nitrone compound has a structure of the following formula, TBN or TN-2: 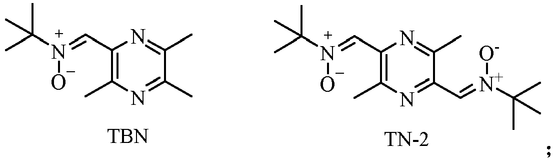
preferably, the SGLT-2 inhibitor the SGLT-2 inhibitor is selected from Dapagliflozin, Canagliflozin, Empagliflozin (Empag), Ipragliflozin, Luseogliflozin, Tofogliflozin, Ertugliflozin, Sergliflozin etabonate, Sotagliflozin, Rongliflozin L-Pyroglutamic Acid, Wangliflozin, Tagliflozin, Jiagliflozin, Henggliflozin, or a combination thereof.

9. Use of a nitrone compound or a pharmaceutically acceptable salt thereof in manufacture of a medicament of an interleukin-6 antagonist;
wherein, the nitrone compound has a structure of the following formula (I): 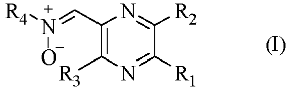 wherein:
R₁ is hydrogen, methyl, or 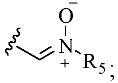
R₂ and R₃ are the same or different, each independently selected from hydrogen and C₁-C₆ alkyl; R₄ is sec-butyl, isobutyl, t-butyl, cyclopentyl or cyclohexyl, and R₅ is sec-butyl, isobutyl, t-butyl, or cyclopentyl;
preferably, R₂ and R₃ are the same or different, each independently selected from hydrogen, methyl, ethyl, n-propyl, isopropyl, n-butyl, isobutyl, tert-butyl, and n-pentyl; R₁ is hydrogen, methyl, or 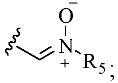
more preferably, the nitrone compound has a structure of the following formula, TBN or TN-2:

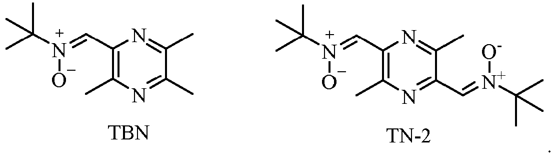

10. Use of tetramethylpyrazine nitrone (TBN) or a pharmaceutically acceptable salt thereof in manufacture of a medicament for the prevention or treatment of chronic kidney disease and a complication thereof, wherein the medicament is an oral drug formulation which comprises a therapeutically effective amount of TBN or a pharmaceutically acceptable salt thereof, the therapeutically effective amount being about 600-2400 mg per day;
wherein, TBN has a structure of the following formula:

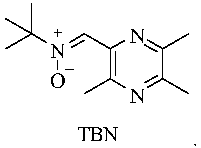

11. The use of claim 10, wherein the therapeutically effective amount is about 600-1200 mg per day.

12. The use of claim 10, wherein the therapeutically effective amount is about 1200-2400 mg per day.

13. The use of any one of claims 10-12, wherein the complication of chronic kidney disease is diabetes complicated with chronic kidney disease, chronic kidney disease complicated with infection, or a combination thereof.

14. The use of any one of claims 10-13, wherein the oral drug formulation further comprises a SGLT-2 being selected from Dapagliflozin, Canagliflozin, Empagliflozin (Empag), Ipragliflozin, Luseogliflozin, Tofogliflozin, Ertugliflozin, Sergliflozin etabonate, Sotagliflozin, Rongliflozin L-Pyroglutamic Acid, Wangliflozin, Tagliflozin, Jiagliflozin, Henggliflozin, or a combination thereof.
